# FASCICULE DE BREVET EUROPEEN

(11) **EP 2 001 483 B1**
(45) Date de publication et mention de la délivrance du brevet: **05.01.2011**
(21) Numéro de dépôt: 07731211.4
(22) Date de dépôt: 28.03.2007
(51) Int. Cl.: A61K 31/575, A61P 9/00, A61P 39/00, A61P 1/16

(54) **UTILISATION DE DERIVES DU CHOLEST-4-EN-3-ONE POUR L'OBTENTION D'UN MEDICAMENT CYTOPROTECTEUR**
VERWENDUNG VON CHOLEST-4-EN-3-ON-DERIVATEN ZUR ERHALTUNG EINES ZYTOPROTEKTIVEN MEDIKAMENTS
USE OF CHOLEST-4-ENE-3-ONE DERIVATIVES TO OBTAIN A CYTOPROTECTIVE MEDICINE

(30) Priorité: 31.03.2006 FR 0602799
(43) Date de publication de la demande: 17.12.2008
(73) Titulaire: Trophos, 13288 Marseille Cedex 9 (FR)
(72) Inventeur: PRUSS, Rebecca, F-13260 Cassis (FR); BUISSON, Bruno, F-13001 Marseille (FR); BORDET, Thierry, F-13009 Marseille (FR)
(74) Mandataire: Galup, Cédric Olivier Nicolas
(86) Numéro de dépôt international: PCT/FR2007/000530
(87) Numéro de publication internationale: WO 2007/118967

(56) Documents cités:
- WO-A-2004/082581
- WO-A-2006/007910
- ALEXANDER-LINDO, RUBY LISA ET AL: "Hypoglycaemic effect of stigmast-4-en-3-one and its corresponding alcohol from the bark of Anacardium occidentale (cashew)" PHYTOTHERAPY RESEARCH , 18(5), 403-407 CODEN: PHYREH; ISSN: 0951-418X, 2004, XP008069051
- SUZUKI K ET AL: "The cholesterol metabolite cholest-4-en-3-one and its 3-oxo derivatives suppress body weight gain, body fat accumulation and serum lipid concentration in mice" BIOORGANIC & MEDICINAL CHEMISTRY LETTERS, OXFORD, GB, vol. 8, no. 16, 18 août 1998 (1998-08-18), pages 2133-2138, XP004137233 ISSN: 0960-894X
- DATABASE WPI Week 199519 Derwent Publications Ltd., London, GB; AN 1995-144712 XP002399332 & JP 07 069898 A (NIPPON OILS & FATS CO LTD) 14 mars 1995 (1995-03-14)
- DATABASE WPI Week 199525 Derwent Publications Ltd., London, GB; AN 1995-190762 XP002399333 & JP 07 109216 A (TSUJI K) 25 avril 1995 (1995-04-25)

## Description

La présente invention concerne l'utilisation de dérivés de cholest-4-èn-3-one, pour l'obtention d'un médicament cytoprotecteur, à l'exception d'un médicament neuroprotecteur.

Les processus dégénératifs cellulaires sont caractérisés par le dysfonctionnement des cellules entraînant souvent des activités cellulaires indésirables et la mort cellulaire.

Les cellules ont développé des mécanismes d'adaptation, en réponse au stress, qui allongent leur durée de vie ou retardent ou empêchent la mort cellulaire (mécanismes cytoprotecteurs).

Cependant, ces mécanismes cytoprotecteurs sont parfois insuffisants, inadéquats, ou induits trop tard pour être efficaces et les cellules meurent. Il peut donc s'avérer intéressant de disposer de nouveaux médicaments, cytoprotecteurs, qui favoriseraient la cytoprotection. C'est un des buts de la présente invention.

Le terme «cytoprotecteur» fait référence à la capacité d'agents, naturels ou non, à protéger une cellule contre la mort cellulaire, particulièrement la mort cellulaire pathologique et/ou contre les dysfonctionnements cellulaires conduisant à la mort cellulaire. Ces dysfonctionnements cellulaires peuvent être par exemple d'origine mitochondriale, comme une réduction de la capacité à générer de l'ATP, une incapacité à capter et/ou retenir le calcium, ou la génération de radicaux libres.

Parmi les mécanismes principaux de mort cellulaire, on distingue essentiellement la nécrose, l'apoptose et la nécroptose.

La nécrose est une mort cellulaire dite "accidentelle" qui survient lors d'un dommage tissulaire. C'est la membrane plasmique de la cellule qui est la plus touchée, entraînant une modification de l'homéostasie de la cellule. Les cellules vont se gorger d'eau au point que cela va entraîner la lyse de leur membrane plasmique. Cette lyse cellulaire conduit au largage dans le milieu environnant du contenu cytoplasmique. La nécrose est à l'origine du processus inflammatoire.

La nécrose peut toucher un ensemble de cellules ou un tissu alors que les autres parties de voisinage restent vivantes. La transformation qui en résulte est une mortification des cellules ou des tissus.

Autrement dit, la nécrose se définit par des modifications morphologiques survenant lorsqu'une cellule arrive en fin de vie à la suite d'événements tels qu'un traumatisme important comme un arrêt ou une diminution de la circulation sanguine au niveau d'un organe, l'hyperthermie (élévation importante de la température), une intoxication par un produit chimique, un choc physique, etc...

Une des nécroses les plus connues est celle du myocarde lors de l'infarctus (arrêt d'apport circulatoire au niveau du muscle cardiaque) due à une oblitération (obstruction) d'une artère coronaire.

L'apoptose fait partie intégrante de la physiologie normale d'un organisme. C'est une forme physiologique de mort cellulaire hautement régulée et elle est nécessaire à la survie des organismes multicellulaires. L'apoptose est un processus qui joue un rôle primordial au cours de l'embryogenèse.

Les cellules en apoptose ou apoptotiques vont s'isoler des autres cellules. L'apoptose implique habituellement des cellules individuelles dans un tissu et ne provoque pas l'inflammation. L'un des points morphologiques caractéristiques de l'apoptose est l'importante condensation à la fois du noyau et du cytoplasme ce qui induit une diminution significative du volume cellulaire. Le noyau se fragmente ensuite, chaque fragment est entouré d'une double enveloppe. Des corps apoptotiques (éléments cytoplasmiques et nucléaires) sont ensuite libérés et vont être absorbés par phagocytose par les cellules voisines.

L'apoptose peut être induite de différentes façons. Par exemple, une radiation, la présence d'un composé chimique ou d'une hormone sont des stimuli susceptibles d'induire une cascade d'événements apoptotiques dans la cellule. Des signaux intracellulaires comme une mitose incomplète ou un dommage à l'ADN peuvent aussi induire l'apoptose.

L'apoptose intervient aussi après l'action d'un génotoxique ou au cours d'une maladie. Certaines pathologies sont caractérisées par une apoptose anormale, entraînant la perte de certaines populations cellulaires, comme par exemple l'hépatotoxicité, les rétinopathies, la cardiotoxicité.

On distingue donc l'apoptose physiologique et l'apoptose pathologique. L'invention s'adresse essentiellement à l'apoptose pathologique.

Il existe d'autres mécanismes de mort cellulaire, comme par exemple la nécroptose, qui présente des caractéristiques de la nécrose et de l'apoptose. Une cellule mourrant par nécroptose présente des caractéristiques similaires à celles d'une cellule mourrant par nécrose, mais les étapes biochimiques de ce mécanisme s'assimilent plus à celles de l'apoptose. Ce mécanisme de mort cellulaire intervient par exemple dans l'ischémie.

C'est donc aussi un des buts de la présente invention que de disposer de nouveaux médicaments qui pourraient permettre de prévenir et/ou traiter la nécrose et/ou l'apoptose pathologique et/ou la nécroptose (médicaments antinécrotiques et/ou antiapoptotiques et/ou antinécroptotiques).

Les processus dégénératifs cellulaires peuvent résulter, entre autres, de situations pathologiques regroupées sous le terme de maladies ou affections dégénératives, de traumatismes, ou d'exposition à divers facteurs.

Ces traumatismes et facteurs peuvent inclure, par exemple, l'exposition aux radiations (UV, gamma), l'hypoxie ou la privation d'oxygène, la privation de nutriments, la privation de facteurs de croissance, des poisons, des toxines cellulaires, des déchets, des toxines environnementales, des radicaux libres, des oxygènes réactifs ou encore certains événements et/ou procédures médicaux comme par exemple les traumatismes chirurgicaux incluant les transplantations de cellules, de tissus et d'organes. On peut citer également des agents chimiques ou biologiques utilisés comme agents thérapeutiques dans le contexte de traitements médicaux comme par exemple des agents cytostatiques ou des agents anti-inflammatoires.

L'invention n'a pas pour but de traiter les causes extracellulaires des pathologies ou des processus dégénératifs pouvant aboutir à la mort cellulaire, mais bien les conséquences au niveau cellulaire desdits processus pathologiques ou des dites pathologies et particulièrement de protéger la cellule contre lesdites conséquences.

Parmi les situations pathologiques caractérisées par un processus dégénératif les plus importantes, autres que les affections neurologiques ou neurodégénératives auxquelles la présente invention ne s'adresse pas, on trouve :
les maladies des os, des articulations, du tissu conjonctif et du cartilage, telles que l'ostéoporose, l'ostéomyélite, les arthrites dont par exemple l'ostéoarthrite, l'arthrite rhumatoïde et l'arthrite psoriatique, la nécrose avasculaire, la fibrodysplasie ossifiante progressive, le rachitisme, le syndrome de Cushing ;
les maladies musculaires telles que la dystrophie musculaire, comme par exemple la dystrophie musculaire de Duchenne, les dystrophies myotoniques, les myopathies et les myasthénies ;
les maladies de la peau, telles que les dermatites, l'eczéma, le psoriasis, le vieillissement, ou encore les altérations de la cicatrisation ;
les maladies cardiovasculaires telles que l'ischémie cardiaque et/ou vasculaire, l'infarctus du myocarde, la cardiopathie ischémique, l'insuffisance cardiaque chronique ou aigue, la dysrythmie cardiaque, la fibrillation auriculaire, la fibrillation ventriculaire, la tachycardie paroxystique, l'insuffisance cardiaque, la cardiomyopathie hypertrophique, l'anoxie, l'hypoxie, les effets secondaires dus à des thérapies avec des agents anticancéreux ;
les maladies circulatoires telles que l'athérosclérose, les scléroses artérielles, et les maladies vasculaires périphériques, les accidents vasculaires cérébraux, les anévrismes ;
les maladies hématologiques et vasculaires telles que : l'anémie, l'amyloïdose vasculaire, les hémorragies, la drépanocytose, le syndrome de la fragmentation des globules rouges, la neutropénie, la leucopénie, l'aplasie médullaire, la pancytopénie, la thrombocytopénie, l'hémophilie ;
les maladies du poumon incluant la pneumonie, l'asthme ; les maladies chroniques obstructives des poumons comme par exemple les bronchites chroniques et l'emphysème ;
les maladies du tractus gastro-intestinal, telles que les ulcères ;
les maladies du foie comme par exemple les hépatites particulièrement les hépatites d'origine virale ou ayant pour agent causal d'autres agents infectieux, les hépatites alcooliques, les hépatites auto-immunes, les hépatites fulminantes, certains désordres métaboliques héréditaires, la maladie de Wilson, les cirrhoses, la maladie hépatique alcoolique (ALD), les maladies du foie dues à des toxines ou des médicaments ; les stéatoses comme par exemple :
   - les stéatose hépatiques non alcooliques (NASH), ou accompagnant l'intoxication exogène à l'alcool, aux médicaments, les hépatites virales ou toxiques, les complications de procédures chirurgicales, les maladies métaboliques (telles que diabète, syndrome d'intolérance au glucose, obésité, hyperlipidémies, dysfonctions de l'axe hypothalamohypophysaire, abétalipoproteinémie, galactosémies, maladies du glycogène, maladie de Wilson, maladie de Weber-Christian, le syndrome de Refsum, déficit en carnitine),
   - les complications hépatiques des maladies inflammatoires du tube digestif,
   - les hépatites auto-immunes.

Par le biais d'une action sur la stéatose ou d'une action sur l'apoptose hépatique quelle qu'en soit la cause les composés pourraient avoir une action préventive sur le développement de la fibrose hépatique et la prévention de la survenue de cirrhoses.les maladies du pancréas comme par exemple les pancréatites aigues ou chroniques ;
les maladies métaboliques telles que les diabètes mellitus et insipide, les thyroïdites ;
les maladies des reins telles que par exemple les désordres rénaux aigus, la toxicité rénale due à des toxines ou des médicaments, la glomérulonéphrite ;
les intoxications sévères par des agents chimiques, des toxines ou des médicaments ;
les affections dégénératives associées au Syndrome Immuno Déficitaire Acquis (SIDA) ;
les désordres associés au vieillissement, tel que le syndrome du vieillissement accéléré ;
les désordres dentaires tels que ceux aboutissant à la dégradation des tissus comme par exemple les périodontites ;
les maladies ou désordres ophtalmiques incluant les rétinopathies diabétiques, le glaucome, le ptosis, l'atrophie optique, l'ophtalmoplégie externe progressive chronique, les dégénérescences maculaires, la dégénérescence rétinienne, la rétinite pigmentaire, les trous ou déchirures rétiniennes, le décollement rétinien, l'ischémie rétinienne, les rétinopathies aigues associées à un traumatisme, les dégénérescences inflammatoires, les complications post chirurgicales, les rétinopathies médicamenteuses, la cataracte ;
les désordres des voies auditives, tels que l'otosclérose et la surdité induite par des antibiotiques ;
les maladies associées aux mitochondries (pathologies mitochondriales), telles que l'ataxie de Friedrich, la dystrophie musculaire congénitale avec anomalie mitochondriale structurelle, certaines myopathies (syndrome des MELAS, syndrome de MERFF, syndrome de Pearson, syndrome de Kearns-Sayre, le syndrome de MIDD (diabètes mitochondriaux et surdité), le syndrome de Wolfram, la dystonie.

L'invention s'intéresse également à la protection des cellules, des tissus et/ou des organes transplantés, que ce soit avant, pendant (prélèvement, transport et/ou réimplantation) ou après la transplantation.

On recherche toujours des composés pharmacologiquement actifs pour lutter contre les processus dégénératifs évoqués ci-dessus.

La présente invention répond à cette demande de composés cytoprotecteurs. En effet, la demanderesse a découvert que les dérivés de cholest-4-èn-3-one, et notamment l'oxime de cholest-4-èn-3-one, sont doués de remarquables propriétés cytoprotectrices.

C'est pourquoi la présente invention a pour objet l'utilisation d'au moins un composé répondant à la formule I 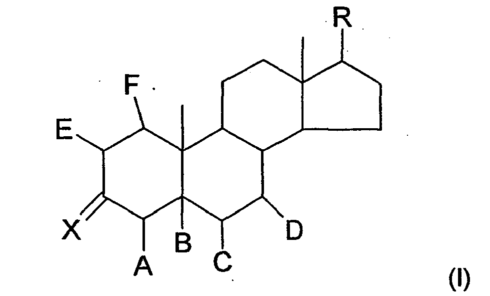 dans laquelle X représente un groupement =N-OH, et R représente un groupement choisi parmi 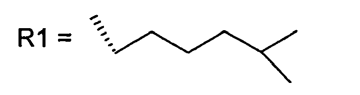 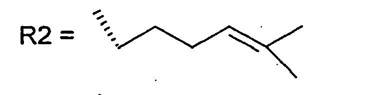 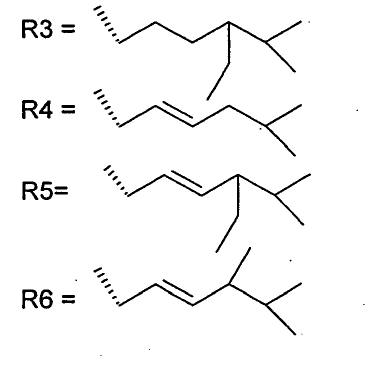
A représente un atome d'hydrogène ou ensemble avec B une liaison carbone-carbone,
B représente un atome d'hydrogène, un groupement hydroxy ou ensemble avec A une liaison carbone-carbone,
C représente un atome d'hydrogène ou ensemble avec D une liaison carbone-carbone,
D représente un atome d'hydrogène ou ensemble avec C une liaison carbone-carbone,
E représente un atome d'hydrogène ou ensemble avec F une liaison carbone-carbone,
F représente un atome d'hydrogène ou ensemble avec E une liaison carbone-carbone,
ou l'un de ses sels d'addition avec les acides pharmaceutiquement acceptables, ou l'un de ses esters ou l'un des sels d'addition avec les acides pharmaceutiquement acceptables desdits esters,
pour la préparation d'un médicament cytoprotecteur, à l'exception d'un médicament neuroprotecteur.

Les composés de formule I tels que définis ci-dessus sont connus (WO2004/082581).

Les sels d'addition avec les acides pharmaceutiquement acceptables peuvent être par exemple des sels formés avec les acides chlorhydrique, bromhydrique, nitrique, sulfurique, phosphorique acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques, ou carboxyliques.

Selon l'invention le groupement oxime représente les isomères syn et anti, purs ou en mélange, associés à l'orientation de la liaison N-O par rapport à la double liaison C=N.

Parmi les composés ci-dessus décrits, on retient plus particulièrement les composés ci-dessus pour lesquels :
- A représente ensemble avec B une liaison carbone-carbone, C, D, représentent un atome d'hydrogène, E, F représentent un atome d'hydrogène ou ensemble une liaison carbone-carbone et R a la signification R1,
- A représente ensemble avec B une liaison carbone-carbone, C, D représentent un atome d'hydrogène, E, F représentent un atome d'hydrogène et R a la signification R2 ou R3 ou R4,
- A représente ensemble avec B une double liaison, C représente ensemble avec D une liaison carbone-carbone, E,F représentent un atome d'hydrogène et R a la signification R1 ou R6,
- A représente ensemble avec B une double liaison, C représente ensemble avec D une liaison carbone-carbone, E représente ensemble avec F, une liaison carbone-carbone et R a la signification R1,
- E représente ensemble avec F une double liaison, C, D, A, B représentent un atome d'hydrogène et R a la signification R1,
ainsi que leurs sels d'addition avec les acides pharmaceutiquement acceptables

Avantageusement, on utilise selon l'invention au moins un composé de formule I choisi parmi
- l'oxime de cholestan-3-one,
- l'oxime de cholest-4-èn-3-one,
- l'oxime de cholest-1,4-dièn-3-one
ou l'un de ses sels d'addition avec les acides pharmaceutiquement acceptables, ou l'un de ses esters ou l'un des sels d'addition avec les acides pharmaceutiquement acceptables desdits esters.

Préférentiellement on utilise selon l'invention l'oxime de cholest-4-èn-3-one ou l'oxime de cholest-1,4-dièn-3-one ou l'un de ses sels d'addition avec les acides pharmaceutiquement acceptables, ou l'un de ses esters ou l'un des sels d'addition avec les acides pharmaceutiquement acceptables desdits esters. Les intéressantes propriétés cytoprotectrices des composés de formule I justifient leur utilisation pour la préparation d'un médicament cytoprotecteur, particulièrement destiné au traitement ou à la prévention de la nécrose et/ou de l'apoptose pathologique et/ou de la nécroptose (médicaments antinécrotiques et/ou antiapoptotiques et/ou antinécroptotiques) ou encore des affections comme
les maladies des os, des articulations, du tissu conjonctif et du cartilage,
les maladies musculaires,
les maladies de la peau,
les maladies cardiovasculaires,
les maladies circulatoires,
les maladies hématologiques et vasculaires,
les maladies du poumon,
les maladies du tractus gastro-intestinal,
les maladies du foie,
les maladies du pancréas,
les maladies métaboliques,
les maladies des reins,
les intoxications sévères,
les affections dégénératives associées au Syndrome Immuno Déficitaire Acquis (SIDA),
les désordres associés au vieillissement,
les désordres dentaires,
les maladies ou désordres ophtalmiques,
les maladies des voies auditives,
les maladies associées aux mitochondries (pathologies mitochondriales).

L'invention s'intéresse également à la protection des cellules, des tissus ou des organes transplantés, que ce soit avant, pendant (prélèvement, transport et/ou réimplantation) ou après la transplantation.

Avantageusement, les composés de formule I peuvent être utilisés dans la préparation d'un médicament destiné à la protection des cellules cardiaques (médicament cardioprotecteur), à la protection des cellules hépatiques (médicament hépatoprotecteur) ou d'un médicament destiné au traitement ou à la prévention des maladies associées aux mitochondries.

Selon l'invention le composé de formule I est avantageusement présent dans le médicament cytoprotecteur à des doses physiologiquement efficaces ; lesdits médicaments renferment notamment une dose cytoprotectrice efficace d'au moins un des composés de formule I.

A titre de médicaments, les composés répondant à la formule I, leurs esters, leurs sels d'addition avec les acides pharmaceutiquement acceptables ainsi que les sels d'additions avec les acides pharmaceutiquement acceptables desdits esters peuvent être formulés pour la voie digestive ou parentérale.

Les médicaments selon l'invention peuvent comprendre en outre au moins un autre ingrédient thérapeutiquement actif, qu'il soit actif sur la même pathologie ou sur une pathologie différente, pour une utilisation simultanée, séparée ou étalée dans le temps, notamment lors d'un traitement chez un sujet atteint de l'une des pathologies précédemment citées.

Selon l'invention, le composé de formule I peut être utilisé dans le médicament, en mélange avec un ou plusieurs excipients ou véhicules inertes, c'est à dire pharmaceutiquement inactifs et non toxiques. On peut citer par exemple des solutions salines, physiologiques, isotoniques, tamponnées, etc., compatibles avec un usage pharmaceutique et connues de l'homme du métier. Les compositions peuvent contenir un ou plusieurs agents ou véhicules choisis parmi les dispersants, solubilisants, stabilisants, conservateurs, etc. Des agents ou véhicules utilisables dans des formulations (liquides et/ou injectables et/ou solides) sont notamment la méthylcellulose, l'hydroxyméthylcellulose, la carboxyméthylcellulose, les cyclodextrines, le polysorbate 80, le mannitol, la gélatine, le lactose, des huiles végétales ou animales, l'acacia, etc. De façon préférentielle, on utilise des huiles végétales. Les compositions peuvent être formulées sous forme de suspension injectable, de gels, huiles, comprimés, suppositoires, poudres, gélules, capsules, etc., éventuellement au moyen de formes galéniques ou de dispositifs assurant une libération prolongée et/ou retardée. Pour ce type de formulation, on utilise avantageusement un agent tel que la cellulose, des carbonates ou des amidons.

L'administration peut être réalisée par toute méthode connue de l'homme du métier, de préférence par voie orale ou par injection, typiquement par voie intra-péritonéale, intra-cérébrale, intra-thécale, intra-veineuse, intra-artérielle ou intra-musculaire. L'administration par voie orale est préférée. S'agissant d'un traitement à long terme, la voie d'administration préférée sera sublinguale, orale ou transcutanée.

Pour les injections, les composés sont généralement conditionnés sous forme de suspensions liquides, qui peuvent être injectées au moyen de seringues ou de perfusions, par exemple, Il est entendu que le débit et/ou la dose injectée, ou de manière générale la dose à administrer, peuvent être adaptés par l'homme du métier en fonction du patient, de la pathologie, du mode d'administration, etc. Il est entendu que des administrations répétées peuvent être réalisées, éventuellement en combinaison avec d'autres ingrédients actifs et/ou tout véhicule acceptable sur le plan pharmaceutique (tampons, solutions saline, isotonique, en présence d'agents stabilisants, etc.).

L'invention est utilisable chez les mammifères, notamment chez l'être humain.

En général la dose journalière du composé sera la dose minimale pour obtenir l'effet thérapeutique souhaité. Les doses des composés ci-dessus décrits et par exemple de l'oxime de cholest-4-èn-3-one seront en général comprises entre 0,001 à 100 mg par kilo-par jour pour l'homme.

Si nécessaire, la dose journalière peut être administrée en deux, trois, quatre, cinq, six ou plus, prises par jour ou par sous-doses multiples administrées par intervalles appropriés pendant la journée.

La quantité choisie dépendra de multiples facteurs, en particulier de la voie d'administration, de la durée d'administration, du moment de l'administration, de la vitesse d'élimination du composé, du ou des différents produits utilisés en combinaison avec le composé, de l'âge, du poids et de la condition physique du patient, ainsi que de son histoire médicale, et de toutes autres informations connues en médecine.

La prescription du médecin traitant pourra commencer à des doses inférieures à celles généralement utilisées, puis ces doses seront progressivement augmentées afin de mieux maîtriser l'apparition d'éventuels effets secondaires.

### Exemple 1 : Effet anti-apoptotique de l'oxime de cholest-4-èn-3-one

Les propriétés antiapoptotiques de l'oxime de cholest-4-èn-3-one ont été analysées sur des cardiomyocytes, par un test de dysfonctionnement contractile induit par la doxorubicine.

Une solution stock de l'oxime de cholest-4-èn-3-one à la concentration de 10mM dans 100% de DMSO a été utilisée. La concentration finale en DMSO a été la même pour tous les points expérimentaux, indépendamment des concentrations en molécules utilisées. L'oxime de cholest-4-èn-3-one a été testé aux concentrations de 0,3, 1 et 3 µM, dilué dans une solution de Tyrode (composition en mmol/L: NaCl 135, KCL 5,4, NaH₂PO₄ 0,33, CaCl₂ 1,2, MgCl₂ 1,0, Hepes 10 ; pH ajusté à 7,4 avec NaOH).

### Méthodes

### Contractilité et apoptose des cardiomyocytes ventriculaires de lapin

### A.1 Obtention de cellules isolées de cardiomyocytes ventriculaires de lapin

Des cellules ventriculaires isolées sont obtenues à partir de coeurs de lapins males de Nouvelle Zélande comme décrit dans A. d'Anglemont de Tassigny et al., Fund Clin Pharmacol, 18: 531-38, 2004. En bref, les lapins (2,0-2,5 kg) sont anesthésiés avec une solution de pentobarbital (50 mg/kg) puis reçoivent de l'héparine (200 IU/kg). Les coeurs sont excisés et immédiatement perfusés, pendant 10 à 15 minutes grâce à un appareil de Langendorff sans recirculation avec une solution isotonique de tyrode (sans calcium) oxygénée (95%, 2-5% CO₂ (en mM: NaCl 135, KCI 5,4, Na₂PO₄ 0,33, MgCl₂ 1,0, HEPES 10, pH ajusté à 7,4 avec NaOH 1 N à 37°C, 280-300 mOsmol/kgH₂O). Ensuite, tous les coeurs sont perfusés pendant 3 minutes en mode "recirculation" avec la même solution de Tyrode sans calcium (débit coronaire, 10-15mL/min) additionné de 1 mg/mL de collagénase type II et 0,28 mg/mL de protéase type XIV. Finalement, tous les coeurs sont perfusés en mode sans recirculation avec la même solution de Tyrode supplémentée avec 0,3 mM CaCl2 pendant 10 min. Le ventricule gauche est enlevé et découpé en petits morceaux, la dissociation cellulaire est réalisée par agitation mécanique douce. Du calcium extracellulaire est ajouté par incrément toutes les 15 minutes, pour arriver à une concentration physiologique de 1,0 mM. Les myocytes isolés sont maintenus dans un milieu sans sérum contenant (en mM) NaCl 110, KCI 5,4, Na₂PO₄ 0,33, NaHCO₃ 25, Glucose 5, MgCl₂ 0,8, CaCl₂ 1, pH ajusté à 7,4 jusqu'à 1 h30 avant l'expérimentation. Toutes les cellules sont en forme de baguette, ont une striation croisée claire et ne présentent pas de vésicule à leur surface au microscope optique.

### A.2 Marquage à l'annexine V

Le marquage à l'annexine V de la phosphatidylsérine a été utilisé comme méthode quantitative de mesure de l'apoptose en utilisant le kit MiniMacs cell isolation (Miltenyi Biotec, Bergisch, Gladbach, Germany). En bref, les cellules exposant de la phosphatidylsérine sont marquées magnétiquement avec des microbilles d'annexine V, puis passées dans une colonne placée dans un champ magnétique. Les cellules marquées (qui présentent de la phosphatidylsérine marquée magnétiquement) sont retenues dans la colonne alors que celles non marquées (cellules nécrotiques et non-apoptotiques) ne sont pas retenues. La colonne est retirée du champ magnétique, les cellules exposant de la phosphatidylsérine retenues magnétiquement sont éluées comme fraction positive et comptées avec une cellule de Mallassez. Le pourcentage de cellules apoptotiques est alors rapporté au nombre initial de cellules.

### A.3 Mesure de l'activité caspase-3

L'activité caspase-3 est utilisée comme méthode quantitative de mesure de l'apoptose. En bref, les cellules sont lysées et le surnageant est utilisé pour la mesure d'activité caspase-3 en utilisant le kit AK-005 (Biomol Research Laboratories, Plymouth Meeting, PA, USA). Le substrat fluorogène pour mesurer l'activité caspase-3 (DEVD) est marqué avec le fluorochrome 7-amino-4-methyl coumarine (AMC) qui produit une fluorescence jaune-verte détectable à la lumière UV à 360/460 nm pendant 210 min. L'AMC est libéré du substrat par clivage par la caspase-3, l'expression de l'enzyme est exprimée en fmol/min.

### A.4 Mesure de la contractilité

Les myocytes sont transférés dans une chambre à 37°C perfusée de façon continue et positionnée sur la platine d'un microscope inversé. La chambre est perfusée avec du tampon physiologique contenant (en mM): NaCl 140 ; KCI 5.4 ; CaCl₂ 1 ; MgCl₂ 0,8 ; HEPES 10 et glucose 5,6 (pH = 7,4 ; 290 mOsmol/kgH₂O).

La contraction des myocytes est induite une fois par seconde (1 Hz) avec des électrodes de champ en platine placées dans la chambre et reliées à un stimulateur. Les images sont saisies de façon continue avec un objectif x 20 et transmises à une caméra CCD à raison de 240 échantillons/s. Les images de la caméra CCD sont projetées sur un écran vidéo.

Les myocytes ont été sélectionnés pour l'étude selon le critère suivant : une apparence en forme de baguette avec des striations bien apparentes et pas de vacuole intracellulaire, pas de contraction spontanée quand on les stimule avec 1 mM Ca²⁺, et avec une longueur de repos et une amplitude de contraction constantes. La longueur des sarcomères a été mesurée grâce à un programme d'analyse d'image vidéo et les données ont été saisies à un rythme de 240 échantillons/s. Les images caméra ont été converties en mesures de longueur de sarcomère. Le pourcentage de contraction est calculé à partir de ces données sur la longueur du sarcomère.

### A.5 Analyse des données

Toutes les données ont été exprimées en moyenne ± écart-type. Les comparaisons des données entre les différents groupes ont été réalisées par ANOVA suivi d'un test de Student avec une différence significative à p<0.05.

### ■ Protocole expérimental

L'apoptose est induite dans les cardiomyocytes isolés par exposition pendant 3 à 8h à 1 µM de doxorubicine ajoutée dans une solution isotonique contenant (en mM) Nacl 110, KCI 5,4, Na₂PO₄ 0,33, NaHCO₃ 25, Glucose 5, MgCl₂ 0,8, CaCl₂ 1, pH ajusté à 7,4. Le marquage à l'annexine V a été réalisé 3 h après le début de l'exposition à la doxorubicine puisque ce phénomène apparaît très tôt dans la cascade apoptotique. Les mesures d'activité caspase-3 sont réalisées 8 h après l'exposition à la doxorubicine puisque ce phénomène a lieu plus tard dans le phénomène d'apoptose. La contractilité des cardiomyocytes a été mesurée toutes les heures pendant les 8 h d'exposition à la doxorubicine. Après tous les traitements, les cellules ont été comparées aux cardiomyocytes contrôles non exposés à la doxorubicine.

Les cardiomyocytes ont été prétraités avec le composé oxime de cholest-4-èn-3-one pendant 15 min avant l'exposition à la doxorubicine. Trois concentrations de ce composé ont été testées lors de cette étude: 0,3 µM, 1 µM et 3 µM.

### ■ Résultats

La longueur moyenne des sarcomères des cellules utilisées dans cette étude n'était pas significativement différente entre les groupes.

### ➢ Effet de la doxorubicine sur la contractilité des myocytes et sur l'apoptose

L'exposition à la doxorubicine a résulté en une diminution au cours du temps du raccourcissement du sarcomère. Le raccourcissement du pic sous doxorubicine était similaire au contrôle pendant les trois premières heures puis devint significativement diminué après 4 h d'exposition (-53,20 ± 7,70% contre - 19,49 ± 2,06% par rapport à la ligne de base de la doxorubicine et du contrôle respectivement, p<0.05, n=5).

Le traitement avec 1 µM de doxorubicine a induit l'apoptose avec une augmentation significative en marquage à l'annexine V et en activité caspase-3.

### ➢ Effet de l'oxime de cholest-4-èn-3-one sur le dysfonctionnement au niveau de la contractilité induite par la doxorubicine et l'apoptose.

Le traitement avec 1 µM de doxorubicine a résulté en une réduction significative du raccourcissement du pic des cardiomyocytes ventriculaires qui est abolie en présence d'oxime de cholest-4-èn-3-one (0,3, 1 et 3 µM). En effet, après 4 h d'exposition, le raccourcissement du pic sous doxorubicine (-53,20 ± 7,70%) devint significativement diminué avec le composé à 0,3 µM (-25,35 ± 0,18%), à 1 µM (-15,66 ± 5,72%) et à 3 µM (-13,95 ± 3,17%) par rapport à la ligne de base.

De plus, les augmentations de marquage à l'annexine V et d'activité caspase-3, dues à la doxorubicine, ont été bloquées par l'oxime de cholest-4-èn-3-one à 0,3, 1 et 3 µM.

L'apoptose évaluée en % de changement en marquage à l'annexine V 3h après doxorubicine donne les résultats suivants : contrôle : 100% ; doxorubicine : 291%± 32 ; doxorubicine + oxime de cholest-4-èn-3-one 0,3 µM : 130%± 12,43 ; doxorubicine + oxime de cholest-4-èn-3-one 1 µM : 121%± 4,74 ; doxorubicine + oxime de cholest-4-èn-3-one 3 µM : 115,5%± 16,35. Les résultats concernant les mesures d'activité caspase-3 sont les suivants : contrôle : 18±9 fmol/min ; doxorubicine : 120±15 fmol/min ; doxorubicine + oxime de cholest-4-èn-3-one 0.3 µM : 33±9 fmol/min ; doxorubicine + oxime de cholest-4-èn-3-one 1 µM : 18±8 fmol/min ; doxorubicine + oxime de cholest-4-èn-3-one 3 µM : 11±4 fmol/min.

### ■ Commentaires et conclusions

Le composé oxime de cholest-4-èn-3-one montre un effet cardioprotecteur sur le dysfonctionnement de contractilité induit par la doxorubicine et sur l'apoptose sur des cardiomyocytes isolés de lapin. La molécule, quand utilisée à des doses appropriées peut effectivement protéger contre la cardiotoxicité induite par la doxorubicine qui est connue comme étant le facteur limitant dans le traitement de patients cancéreux avec cette anthracycline. Ainsi, le composé oxime de cholest-4-èn-3-one pourrait être utilisé pour limiter la cardiotoxicité de la doxorubicine chez ces patients.

### Exemple 2 : Effet de l'oxime de cholest-4-èn-3-one dans un modèle in vivo d'hépatotoxicité aigu.

Les hépatocytes comme de nombreuses autres cellules portent le récepteur Fas/CD95 sur leur membrane cytoplasmique. La stimulation de cette voie Fas induit la mort cellulaire en activant la cascade des caspases.

Un modèle aigu de dommage hépatique peut être induit par une injection unique de l'anticorps anti-Fas Jo2 (Ogasawara et al., Nature, août 1993), produisant des dommages hépatiques sévères et ressemblant à l'hépatite virale, autoimmune ou induite par des drogues.

L'Alanine Aminotransferase (ALAT) aussi appelée la Serum Glutamic Pyruvic Transaminase sérique (SGPT) est une enzyme présente dans les hépatocytes. Son activité augmente de façon importante dans le plasma après lyse hépatique et est donc un bon marqueur pour évaluer le dommage hépatique.

L'expérience conduite a consisté en l'intoxication par Jo2 des animaux suivie d'un dosage des ALAT et à tester la capacité de l'oxime de cholest-4-èn-3-one à protéger les hépatocytes.

### Matériels et Méthodes

### Animaux

Des souris adultes CD1 mâles en provenance de "Elevage Janvier", (Le Genest-Saint-Isle, France) ont été utilisées. Les animaux ont été identifiés individuellement et ont eu libre accès à l'eau et à la nourriture.

Les installations ont été maintenues à un cycle contrôlé de lumière (7:00 - 19:00), et à des températures de 20 ± 2 °C et d'humidité de 50 ± 20 %.

### Préparation de l'anticorps Jo2

La solution stock d'anticorps monoclonal de hamster anti-souris CD95 (Fas), appelé Jo2, provenant de Pharmingen (BD Biosciences, ref. 554254 batch 66081) est à la concentration de 1 mg/mL dans de l'eau. Les dilutions utilisées sont réalisées dans du chlorure de sodium à 0,9% dans de l'eau.

### Préparation du composé à tester

La quantité désirée d'oxime de cholest-4-èn-3-one est pesée et broyée en une fine poudre, puis mise en suspension à 60 mg/ml dans de l'huile de maïs. Le composé est administré par voie orale (par gavage) à une concentration de 5 ml/kg.

### Protocole

Un prétraitement par l'oxime de cholest-4-èn-3-one est réalisé à la dose de 300 mg/kg par administration orale 4h avant l'administration de l'anticorps Jo2. L'anticorps Jo2 est administré par injection intrapéritonéale à la dose de 125 µg/kg dans un volume de 5 mL/kg de poids corporel.

Un contrôle est réalisé sur des animaux recevant un prétraitement par administration orale 4h avant l'administration de l'anticorps d'un volume identique d'huile de maïs ayant été utilisée pour la préparation du composé à tester, sans composé.

### Dosage des ALAT

Du sang des souris anesthésiées est prélevé 24h après administration de Jo2. Le dosage des ALAT est réalisé en utilisant un kit (Roche Diagnostics) avec un spectrophotomètre (Hitachi Modular), selon la méthode standardisée par l'IFCC (Fédération Internationale de Chimie Clinique).

### Résultats et conclusions

L'administration intrapéritonéale de Jo2 à 125 µg/kg a induit la mort de 3 souris sur 19 dans le groupe contrôle dans les 24h suivant l'injection.

L'activité ALAT est significativement réduite par le composé testé à 300 mg/kg.

**Table 1 : Activité ALAT mesurée 24h après administration de Jo2**

| Traitement | Activité ALAT (U/L) Moyenne +/- SEM (n) |
|---|---|
| Contrôle | 2586 ± 474 (16) |
| l'oxime de cholest-4-èn-3-one (300 mg/kg) | 1136 ± 175 (20) ** |

| | |
|---|---|
| ** : p=0:0037, t-test effectué par rapport au groupe contrôle | |

L'oxime de cholest-4-èn-3-one administré à 300 mg/kg, 4h avant l'anticorps Jo2, a permis de :
- empêcher la mort de souris recevant l'anticorps Jo2 dans les 24h suivant l'injection;
- limiter la mort cellulaire induite par une dose sublétale d'anticorps ; L'activité ALAT, biomarqueur de la cytolyse hépatique dans le plasma, est significativement plus basse chez les souris traitées par l'oxime de cholest-4-èn-3-one que chez les souris contrôles non traitées.

### Conclusions

Le modèle d'hépatotoxicité aigüe induite chez la souris par un anticorps anti Fas (Jo2) a permis de mettre en évidence les propriétés hépatoprotectrices de l'oxime de cholest-4-èn-3-one.

Ces effets remarquables permettent d'envisager pour les composés de formule I une utilisation dans la préparation d'un médicament cytoprotecteur en général.

### Etude toxicologique

L'administration chez la souris, en particulier de l'oxime de cholest-4-èn-3-one, par les voies orale, sous-cutanée, intra-péritonéale et intra-veineuse, à des doses allant jusqu'à 300 mg/kg/jour, par traitement avec administration quotidienne pouvant aller jusqu'à 28 jours, n'a pas montré de toxicité significative.

Chez le singe, l'administration par voie orale de doses croissantes journalières jusqu'à 1500 mg/kg sur une période de 10 jours n'a révélé aucune toxicité.

## Revendications

1. Utilisation d'au moins un composé répondant à la formule I 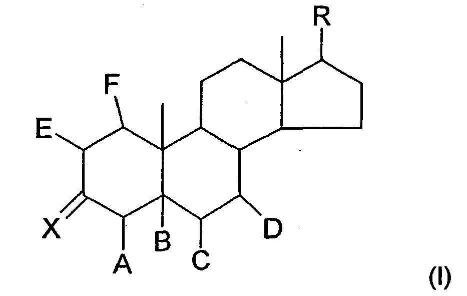 dans laquelle X représente un groupement =N-OH, et R représente un groupement choisi parmi 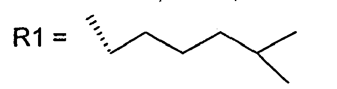 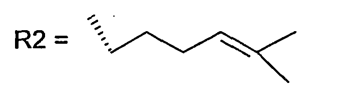 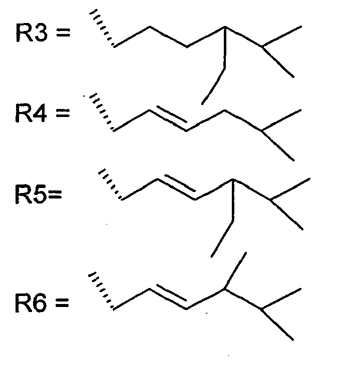 A représente un atome d'hydrogène ou ensemble avec B une liaison carbone-carbone,
B représente un atome d'hydrogène, un groupement hydroxy ou ensemble avec A une liaison carbone-carbone,
C représente un atome d'hydrogène ou ensemble avec D une liaison carbone-carbone,
D représente un atome d'hydrogène ou ensemble avec C une liaison carbone-carbone,
E représente un atome d'hydrogène ou ensemble avec F une liaison carbone-carbone,
F représente un atome d'hydrogène ou ensemble avec E une liaison carbone-carbone,
ou l'un de ses sels d'addition avec les acides pharmaceutiquement acceptables, ou l'un de ses esters ou l'un des sels d'addition avec les acides pharmaceutiquement acceptables desdits esters,
pour l'obtention d'un médicament cytoprotecteur, à l'exception d'un médicament neuroprotecteur.

2. Utilisation selon la revendication 1, **caractérisée en ce que** dans la formule I, A représente ensemble avec B une liaison carbone-carbone, C, D, représentent un atome d'hydrogène, E, F représentent un atome d'hydrogène ou ensemble une liaison carbone-carbone et R a la signification R1.

3. Utilisation selon la revendication 1, **caractérisée en ce que** dans la formule I, A représente ensemble avec B une liaison carbone-carbone, C, D représentent un atome d'hydrogène, E, F représentent un atome d'hydrogène et R a la signification R2 ou R3 ou R4.

4. Utilisation selon la revendication 1, **caractérisée en ce que** dans la formule I, A représente ensemble avec B une double liaison, C représente ensemble avec D une liaison carbone-carbone, E, F représentent un atome d'hydrogène et R a la signification R1 ou R6.

5. Utilisation selon la revendication 1, **caractérisée en ce que** dans la formule I, A représente ensemble avec B une double liaison, C représente ensemble avec D une liaison carbone-carbone, E représente ensemble avec F une liaison carbone-carbone, et R a la signification R1.

6. Utilisation selon la revendication 1, **caractérisée en ce que** dans la formule I, E représente ensemble avec F une double liaison, C, D, A, B représentent un atome d'hydrogène et R a la signification R1.

7. Utilisation selon la revendication 1, **caractérisée en ce que** le composé de formule I est choisi parmi
- l'oxime de cholestan-3-one
- l'oxime de cholest-4-èn-3-one,
- l'oxime de cholest-1,4-dièn-3-one,
ou l'un de ses sels d'addition avec les acides pharmaceutiquement acceptables, ou l'un de ses esters ou l'un des sels d'addition avec les acides pharmaceutiquement acceptables desdits esters.

8. Utilisation selon la revendication 1, **caractérisée en ce que** le composé de formule I est choisi parmi l'oxime de cholest-4-èn-3-one ou l'oxime de cholest-1,4-dièn-3-one ou l'un de ses sels d'addition avec les acides pharmaceutiquement acceptables, ou l'un de ses esters ou l'un des sels d'addition avec les acides pharmaceutiquement acceptables desdits esters.

9. Utilisation selon l'une quelconque des revendications précédentes, **caractérisée en ce que** le médicament est destiné au traitement ou à la prévention de la nécrose et/ou de l'apoptose pathologique et/ou de la nécroptose (médicaments antinécrotiques et/ou antiapoptotiques et/ou antinécroptotique) ou encore des affections comme
les maladies des os, des articulations, du tissu conjonctif et/ou du cartilage ;
les maladies musculaires ;
les maladies de la peau ;
les maladies cardiovasculaires ;
les maladies circulatoires ;
les maladies hématologiques et vasculaires ;
les maladies du poumon ;
les maladies du tractus gastro-intestinal ;
les maladies du foie ;
les maladies du pancréas ;
les maladies métaboliques ;
les maladies des reins ;
les intoxications sévères ;
les affections dégénératives associées au Syndrome Immuno Déficitaire Acquis (SIDA) ;
les désordres associés au vieillissement ;
les désordres dentaires ;
les maladies ou désordres ophtalmiques ;
les maladies des voies auditives ;
les maladies associées aux mitochondries (pathologies mitochondriales).

10. Utilisation selon l'une quelconque des revendications précédentes, **caractérisée en ce que** le médicament est destiné à la protection des cellules cardiaques (médicament cardioprotecteur).

11. Utilisation selon l'une quelconque des revendications 1 à 9 **caractérisée en ce que** le médicament est destiné à la protection des cellules hépatiques (médicament hépatoprotecteur).

12. Utilisation selon l'une quelconque des revendications 1 à 9, **caractérisée en ce que** le médicament est destiné au traitement ou à la prévention des maladies associées aux mitochondries.

13. Utilisation selon l'une quelconque des revendications 1 à 9, **caractérisée en ce que** le médicament est destiné à la protection de cellules, d'un tissu ou d'un organe, avant, pendant ou après une transplantation.

## Claims

1. Use of at least one compound corresponding to formula I 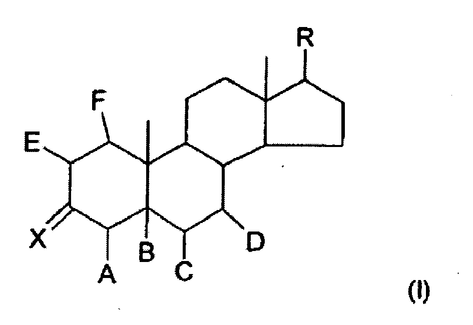 in which X represents an =N-OH group, and R represents a group chosen from 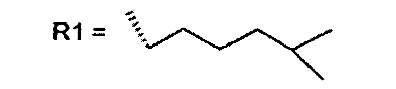 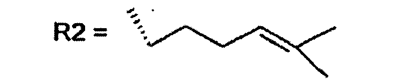 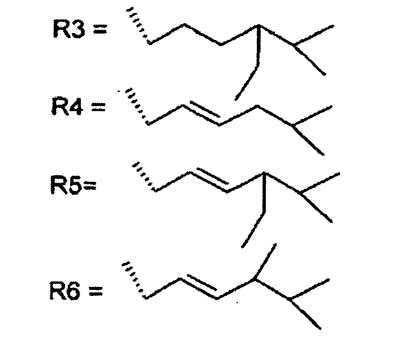 A represents a hydrogen atom or together with B, a carbon-carbon bond,
B represents a hydrogen atom, a hydroxy group or together with A, a carbon-carbon bond,
C represents a hydrogen atom or together with D, a carbon-carbon bond,
D represents a hydrogen atom or together with C, a carbon-carbon bond,
E represents a hydrogen atom or together with F, a carbon-carbon bond,
F represents a hydrogen atom or together with E, a carbon-carbon bond,
or one of its addition salts with pharmaceutically acceptable acids, or one of its esters or one of the addition salts with pharmaceutically acceptable acids of said esters,
in order to obtain a cytoprotective medicament, with the exception of a neuroprotective medicament.

2. Use according to claim 1, **characterized in that** in formula I, A represents together with B, a carbon-carbon bond, C, D, represent a hydrogen atom, E, F represent a hydrogen atom or together a carbon-carbon bond and R has the meaning R1.

3. Use according to claim 1, **characterized in that** in formula I, A represents together with B, a carbon-carbon bond, C, D, represent a hydrogen atom, E, F represent a hydrogen atom and R has the meaning R2 or R3 or R4.

4. Use according to claim 1, **characterized in that** in formula I, A represents together with B, a double bond, C represents together with D, a carbon-carbon bond, E, F represent a hydrogen atom and R has the meaning R1 or R6.

5. Use according to claim 1, **characterized in that** in formula I, A represents together with B, a double bond, C represents together with D, a carbon-carbon bond, E represents together with F, a carbon-carbon bond and R has the meaning R1.

6. Use according to claim 1, **characterized in that** in formula I, E represents together with F, a double bond, C, D, A, B represent a hydrogen atom and R has the meaning R1.

7. Use according to claim 1, **characterized in that** the compound of formula Is selected from
- cholestan-3-one oxime,
- cholest-4-en-3-one oxime,
- cholest-1,4-dien-3-one oxime,
or one of its addition salts with pharmaceutically acceptable acids, or one of its esters or one of the addition salts with pharmaceutically acceptable acids.of said esters.

8. Use according to claim 1, **characterized in that** the compound of formula I is selected from cholest-4-en-3-one oxime or cholest-1,4-dien-3-one oxime or one of its addition salts with pharmaceutically acceptable acids, or one of its esters or one of the addition salts with pharmaceutically acceptable acids of said esters.

9. Use according to any one of the previous claims, **characterized in that** the medicament is intended for the treatment or the prevention of necrosis and/or pathological apoptosis and/or necroptosis (anti-necrotic and/or anti-apoptotic and/or anti-necroptotic medicaments) or further diseases such as
diseases of bones, joints, connective tissue and/or cartilage;
muscular diseases;
skin diseases,
cardiovascular diseases;
circulatory diseases;
haematological and vascular diseases;
diseases of lung;
diseases of gastrointestinal tract;
diseases of liver;
diseases of pancreas;
metabolic diseases;
diseases of kidneys;
severe intoxications;
degenerative diseases associated with Acquired Immune Deficiency Syndrome (AIDS);
disorders associated with aging;
dental disorders;
ophthalmic diseases or disorders;
diseases of auditory pathways;
diseases associated with mitochondria (mitochondrial pathologies).

10. Use according to any one of the previous claims, **characterized in that** the medicament is intended for the protection of cardiac cells (cardioprotective medicament).

11. Use according to any one of claims 1 to 9, **characterized in that** the medicament is intended for the protection of hepatic cells (hepatoprotective medicament).

12. Use according to any one of claims 1 to 9, **characterized in that** the medicament is intended for the treatment or prevention of diseases associated with mitochondria.

13. Use according to any one of claims 1 to 9, **characterized in that** the medicament is intended for the protection of cells, a tissue or an organ, before, during or after a transplant.

## Patentansprüche

1. Verwendung wenigstens einer Verbindung der Formel I 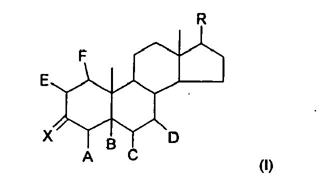 wobei X eine Gruppierung =N-OH repräsentiert
und R eine Gruppierung repräsentiert, die ausgewählt ist aus 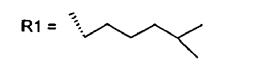 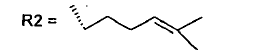 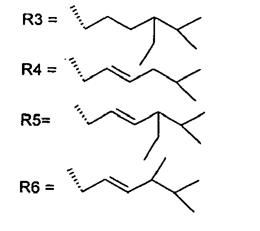 A ein Wasserstoffatom oder zusammen mit B eine Kohlenstoff-Kohlenstoff-Bindung repräsentiert,
B ein Wasserstoffatom, eine Hydroxylgruppe oder zusammen mit A eine Kohlenstoff-Kohlenstoff-Bindung repräsentiert,
C ein Wasserstoffatom oder zusammen mit D eine Kohlenstoff-Kohlenstoff-Bindung repräsentiert,
D ein Wassserstoffatom oder zusammen mit C eine Kohlenstoff-Kohlenstoff-Bindung repräsentiert,
E ein Wassserstoffatom oder zusammen mit F eine Kohlenstoff-Kohlenstoff-Bindung repräsentiert,
F ein Wassserstoffatom oder zusammen mit E eine Kohlenstoff-Kohlenstoff-Bindung repräsentiert,
oder eines seiner Additionssalze mit pharmazeutisch verträglichen Säuren oder eines seiner Ester oder eines seiner Additionssalze mit pharmazeutisch verträglichen Säuren der genannten Ester, zum Erzeugen eines zytoprotektiven Medikaments, mit Ausnahme eines neuroprotektiven Medikaments.

2. Verwendung nach Anspruch 1, **dadurch gekennzeichnet, dass** in Formel I A zusammen mit B eine Kohlenstoff-Kohlenstoff-Bindung repräsentiert, C, D ein Wasserstoffatom repräsentieren, E, F ein Wasserstoffatom oder zusammen eine Kohlenstoff-Kohlenstoff-Bindung repräsentieren und R die Bedeutung R1 hat.

3. Verwendung nach Anspruch 1, **dadurch gekennzeichnet, dass** in Formel I A zusammen mit B eine Kohlenstoff-Kohlenstoff-Bindung repräsentiert, C, D ein Wasserstoffatom repräsentieren, E, F ein Wasserstoffatom repräsentieren und R die Bedeutung R2 oder R3 oder R4 hat.

4. Verwendung nach Anspruch 1, **dadurch gekennzeichnet, dass** in Formel I A zusammen mit B eine Doppelbindung repräsentiert, C zusammen mit D eine Kohlenstoff-Kohlenstoff-Bindung repräsentiert, E, F ein Wasserstoffatom repräsentieren und R die Bedeutung R1 oder R6 hat.

5. Verwendung nach Anspruch 1, **dadurch gekennzeichnet, dass** in Formel I A zusammen mit B eine Doppelbindung repräsentiert, C zusammen mit D eine Kohlenstoff-Kohlenstoff-Bindung repräsentiert, E zusammen mit F eine Kohlenstoff-Kohlenstoff-Bindung repräsentiert und R die Bedeutung R1 hat.

6. Verwendung nach Anspruch 1, **dadurch gekennzeichnet, dass** in Formel I E zusammen mit F eine Doppelbindung repräsentiert, C, D, A, B ein Wasserstoffatom repräsentieren und R die Bedeutung R1 hat.

7. Verwendung nach Anspruch 1, **dadurch gekennzeichnet, dass** die Verbindung der Formel I ausgewählt ist aus:
- Cholestan-3-on-oxim
- Cholest-4-en-3-on-oxim,
- Cholest-1,4-dien-3-on-oxim,
oder einem seiner Additionssalze mit pharmazeutisch verträglichen Säuren oder einem seiner Ester oder einem der Additionssalze mit pharmazeutisch verträglichen Säuren der genannten Ester.

8. Verwendung nach Anspruch 1, **dadurch gekennzeichnet, dass** die Verbindung der Formel I ausgewählt ist aus Cholest-4-en-3-on-oxim oder Cholest-1,4-dien-3-on-oxim oder einem seiner Additionssalze mit pharmazeutisch verträglichen Säuren oder einem seiner Ester oder einem der Additionssalze mit pharmazeutisch verträglichen Säuren der genannten Ester.

9. Verwendung nach einem der vorherigen Ansprüche, **dadurch gekennzeichnet, dass** das Medikament bestimmt ist zur Behandlung oder Prävention von Nekrose und/oder pathologischer Apoptose und/oder Nekroptose (antinekrotische und/oder antiapoptotische und/oder antinekroptotische Medikamente) oder auch Leiden wie Erkrankungen der Knochen, der Gelenke, des Bindegewebes und/oder des Knorpels;
Muskelerkrankungen;
Hauterkrankungen;
kardiovaskuläre Erkrankungen;
Kreislauferkrankungen;
hämatologische und vaskuläre Erkrankungen,
Lungenerkrankungen;
Erkrankungen des Gastrointestinaltrakts;
Lebererkrankungen;
Pankreaserkrankungen;
Stoffwechselerkrankungen;
Nierenerkrankungen;
schwere Intoxikationen;
degenerative Erkrankungen in Verbindung mit dem erworbenen Immundefizienzsyndrom (AIDS);
mit dem Altern verbundene Störungen;
dentale Störungen;
ophthalmische Erkrankungen oder Störungen; Hörwegserkrankungen;
Krankheiten in Verbindung mit den Mitochondrien (mitochondriale Pathologien).

10. Verwendung nach einem der vorherigen Ansprüche, **dadurch gekennzeichnet, dass** das Medikament zum Schützen der Herzzellen (kardioprotektives Medikament) bestimmt ist.

11. Verwendung nach einem der Ansprüche 1 bis 9, **dadurch gekennzeichnet, dass** das Medikament zum Schützen der Leberzellen (hepatoprotektives Medikament) bestimmt ist.

12. Verwendung nach einem der Ansprüche 1 bis 9, **dadurch gekennzeichnet, dass** das Medikament zur Behandlung oder Prävention von Krankheiten in Verbindung mit den Mitochondrien bestimmt ist.

13. Verwendung nach einem der Ansprüche 1 bis 9, **dadurch gekennzeichnet, dass** das Medikament zum Schützen von Zellen, eines Gewebes oder Organs vor, während oder nach einer Transplantation bestimmt ist.
